# EUROPÄISCHE PATENTSCHRIFT

(11) **EP 2 314 205 B1**
(45) Veröffentlichungstag und Bekanntmachung des Hinweises auf die Patenterteilung: **25.05.2016**
(21) Anmeldenummer: 09405185.1
(22) Anmeldetag: 23.10.2009
(51) Int. Cl.: A61B 3/13, A61B 3/135

(54) **Augenuntersuchungsgerät**
Device for examining eyes
Appareil d'examen des yeux

(43) Veröffentlichungstag der Anmeldung: 27.04.2011
(73) Patentinhaber: HAAG-STREIT AG, 3098 Köniz (CH)
(72) Erfinder: Walker, Jürg, 3054 Schüpfen (CH)
(74) Vertreter: Rüfenacht, Philipp Michael

(56) Entgegenhaltungen:
- WO-A1-2005/044098
- WO-A1-2008/136779
- WO-A2-2008/106590
- JP-A- 2008 034 385
- US-A- 5 488 443
- US-A1- 2005 024 587
- US-A1- 2005 063 194
- US-A1- 2006 139 572

## Beschreibung

Die Erfindung betrifft eine Vorrichtung zur Untersuchung und/oder Behandlung eines Auges, mit einer Versorgungsleitung und einem Modul, welches zur Versorgung mit elektrischer Energie mit der Versorgungsleitung verbunden ist.

### Stand der Technik

Spaltlampenmikroskope sind ophthalmologische Untersuchungsgeräte, mit welchen die Augen stereoskopisch untersucht werden können. Bekannte Spaltlampenmikroskope haben zur stereoskopischen Betrachtung eines Auges eine Betrachtungseinheit sowie eine Beleuchtungseinheit. Die Beleuchtungseinheit ist auf einem vertikal verlaufenden Ast einer Halteeinheit angeordnet, wobei die Bedienelemente zur Steuerung der Beleuchtungseinheit im Bereich der Beleuchtungseinheit selber angebracht sind. Das zu betrachtende Auge ist in einer annähernd horizontal verlaufenden Ebene auf einer Seite der Halteeinheit positionierbar. Die Beleuchtungseinheit umfasst eine Glühlampe für eine Spaltbeleuchtung, wobei die Helligkeit der Glühlampe einstellbar ist. Der Querschnitt des von der Beleuchtungseinheit ausgehenden Strahls zur Augenbeleuchtung (Spalt) ist in dessen Bereich einstellbar. Das Spaltlampenmikroskop weist weiter eine Fixierlampe und eine Umfeldbeleuchtung auf. Die Schrift US 5488443 beschreibt z.B. ein Spaltlampenmikroskop gemäss dem Oberbegriff des Anspruchs 1.

Bei den gegenwärtigen Vorrichtungen zur Untersuchung und/oder Behandlung eines Auges (nachfolgend Untersuchungsvorrichtung genannt) bestehen folgende Nachteile:
1. Die Bedienelemente sind nicht ergonomisch platziert, sondern konstruktionsbedingt im Bereich der zu steuernden Funktion untergebracht. 2. Während einer Untersuchung ist der Untersuchende mit der Betrachtung des Auges beschäftigt und muss für Justierungen der Untersuchungsvorrichtung in der Regel blind nach den Bedienelementen suchen.
3. Gewisse Bedienelemente bewegen sich bei deren Verstellung relativ zueinander, womit ein blindes Auffinden der Bedienelemente während der Untersuchung erschwert wird. 4. Der mit der Betrachtung des Auges beschäftigte Untersuchende läuft Gefahr auf der Suche eines Bedienelements ein solches oder einen anderen Gegenstand in nicht beabsichtigter Weise zu betätigen, womit der zu Untersuchende geschädigt werden könnte.
5. Ungeschulte Benutzer benötigen viel Zeit, bis sie mit den über die gesamte Vorrichtung zerstreuten Bedienelementen zurechtkommen, womit insbesondere auch eine Schulung aufwendiger wird.

### Darstellung der Erfindung

Aufgabe der Erfindung ist es, eine dem eingangs genannten technischen Gebiet zugehörende Vorrichtung zur Untersuchung eines Auges zu schaffen, welche einfach aufgebaut und ergonomisch in der Bedienung sowie preislich günstig herstellbar ist.

Die Lösung der Aufgabe ist durch die Merkmale des Anspruchs 1 definiert. Gemäss der Erfindung ist das Modul mit einer an die Versorgungsleitung angeschlossenen Kommunikationsvorrichtung zum Senden und/oder Empfangen von Daten über die Versorgungsleitung verbunden.

Das Modul umfasst eine Kommunikationsvorrichtung, mit dem Daten über die Versorgungsleitung empfangen und/oder gesendet werden können. Die Vorrichtung kann auch mehrere Module umfassen, welche jeweils eine Kommunikationsvorrichtung enthalten und über die Versorgungsleitung miteinander verbunden sind. Damit können die Module mittels ihrer Kommunikationsvorrichtungen Daten über die Versorgungsleitung untereinander austauschen.

Dadurch wird erreicht, dass ein Modul nicht mehr durch lokal zu betätigende Bedienelemente betätigt, d. h. gesteuert werden muss, sondern dass die Informationen zur Steuerung des Moduls über die Versorgungsleitung zu der mit dem Modul verbundenen Kommunikationsvorrichtung und damit zum Modul übertragen werden können. Die Module sind hierbei derart ausgebildet, dass dessen Funktion(en) nicht oder nicht nur durch manuelle Betätigung entsprechender Bedienelemente am Modul selber, sondern durch Übermittlung entsprechender Steuerdaten an das Modul erfolgen kann. Die Erzeugung der Steuerdaten kann dann an einem anderen Ort erfolgen, an dem die benötigten Bedienelemente für den Untersuchenden ergonomisch günstig positioniert werden können. Von dort aus können die Steuerdaten dann über die Versorgungsleitung zu dem Modul übertragen werden. Da sich die Position der Bedienelemente auf diese Weise auch nicht gegenseitig ändern, wird nicht nur das blinde Auffinden der Bedienelemente während der Untersuchung, sondern auch die Schulung von Benutzern deutlich vereinfacht. Ebenso ist die Gefahr, dass ein Bedienelement oder ein anderer Gegenstand unbeabsichtigt betätigt wird, eliminiert oder zumindest verringert.

Ein weiterer Vorteil der Erfindung ergibt sich dadurch, dass für die Übertragung von Daten von bzw. zu einem Modul keine zusätzlichen Leitungen vorgesehen sein müssen sondern diejenigen Leitungen Verwendung finden können, welche zur Versorgung des Moduls mit elektrischer Energie vorgesehen sind. Damit kann der Aufbau der Vorrichtung zur Untersuchung eines Auges besonders einfach und kostengünstig gehalten werden. Weiter wird damit eine Fehlerquelle vermindert, da weniger Leitungen defekt, insbesondere Kabelbrüche erleiden können. Dies ist vor allem bei Geräten von Vorteil, welche mehrere sich relativ zueinander bewegende Teile aufweisen, die durch diese Kabel verbunden sind.

Des Weiteren ergibt sich damit der Vorteil, dass herkömmliche Vorrichtungen einfach nachrüstbar sind, wiederum da keine zusätzlichen Leitungen verbaut werden müssen. Die typischen zweipoligen Kabelverbindungen zwischen Speisegerät und Beleuchtungsoberteil bei einem Spaltlampenmikroskop können beibehalten und die Module daran angeschlossen werden. Damit wird weiter ein einfaches Umrüsten im Feld ermöglicht (Retrofit).

Vorzugsweise sind die Daten in Form eines elektrischen Signals mit einer Frequenz grösser als 100 Hz über die Versorgungsleitung übertragbar, wobei die Frequenz insbesondere höher als 1 kHz, vorzugsweise im Bereich von 50 kHz bis 2 MHz ist. Da die Bandbreite der Datenübertragung im Wesentlichen proportional zur Frequenz der zu übertragenden elektrischen Signale ist, wird die Frequenz bevorzugt hoch gewählt. Grundsätzlich wäre aber auch eine Datenübertragung mit niedrigeren Bitraten, d. h. niedrigen Frequenzen im Bereich von wenigen Hundert Hz bis einigen Tausend Herz möglich. Falls die Datenübertragung über eine Netzstromleitung erfolgt, welche typischerweise mit Wechselstrom von 50 Hz betrieben wird, sollten die Daten mit mindestens 100 Hz übertragen werden, damit Fehler bei der Übertragung vermieden werden können. Auch eine Übertragung mit Frequenzen über 2 MHz wäre theoretisch möglich, allerdings steigt die Signaldämpfung mit der Frequenz, weshalb zusätzliche Massnahmen für eine genügende Signalqualität notwendig sein könnten. Auch EMV Probleme können bei solch hohen Frequenzen vermehrt auftreten.

Die Kommunikationsvorrichtung umfasst bevorzugt einen Koppler, mit welchem Daten in die Versorgungsleitung ein- und/oder aus der Versorgungsleitung auskoppelbar sind. Dabei können Module, welche einen Sensor oder ähnliches umfassen, erfasste Daten in die Versorgungsleitung einkoppeln, während Module, welche Daten zum Beispiel mittels einer Vorrichtung verarbeiten können, Daten aus der Versorgungsleitung auskoppeln können. Weiter können Module aufgrund empfangener Daten dieselben oder weitere Daten an weitere Module senden, womit solche Module sowohl Daten empfangen und senden können, indem die Daten in die Versorgungsleitung eingekoppelt und/oder aus der Versorgungsleitung ausgekoppelt werden. Der Koppler kann dabei als PLC (Powerline Communication) Einheit ausgebildet sein. Insbesondere kann der Koppler zum Beispiel nach Cenelec-Norm, insbesondere nach Cenelec-Norm Band C mit einer Frequenz von 132.5 kHz und einer Baud-Rate von 4800 bps, realisiert sein.

Die Datenübertragung kann mit verschiedenen Übertragungstechniken wie etwa Amplituden-, Phasen- oder Frequenzmodulationen erfolgen. Vorzugsweise erfolgt die Datenübertragung über die Versorgungsleitung mittels FSK (Frequency Shift Keying oder Frequenzumtastung), wobei auch verschiedene Arten von FSK wie etwa GMSK (Gaussian Minimum Shift Keying) möglich sind. Die Daten können mittels eines ersten Moduls über eine RS-232 Schnittstelle empfangen und per FSK in die Versorgungsleitung eingekoppelt werden, wobei das übertragene FSK Signal von einem zweiten Modul aus der Versorgungsleitung wieder ausgekoppelt und über eine RS-232 Schnittstelle ausgegeben werden können. Anstelle von RS-232 könnte auch irgend ein anderer Standard für die Datenübertragung zwischen zwei Geräten verwendet werden. Es ist natürlich auch möglich, von einem bestehenden Standard nur einzelne oder mehrere Aspekte wie das Timing, die Spannungspegel, das Protokoll oder die Definition der physikalischen Schnittstelle, d.h. des Steckers zu verwenden. Durch die Verwendung der einfachen, bekannten, weit verbreiteten und häufig genutzten RS-232 Schnittstelle wird allerdings erreicht, dass die Daten direkt von einem PC (Personal Computer) gelesen und gegebenenfalls weiter verarbeitet werden können. So kann zum Beispiel automatisch ein Bericht anhand der Daten erstellt werden, womit dem Untersuchenden Arbeit erspart werden kann. Natürlich können auch andere als das binäre System mittels FSK Verwendung finden, wobei aber entsprechend viele Frequenzen unterschieden werden müssen. Statt RS-232 Schnittstellen können auch USB Schnittstellen, Bluetooth oder weitere dem Fachmann bekannte Schnittstellen vorgesehen sein.

Die Auskopplung der Daten kann zum Beispiel mittels Hochpass-Filter erfolgen, welche nur Frequenzen durchlassen, welche über einer bestimmten Grenzfrequenz liegen. Mit einem Tiefpass-Filter, welche eine zum Hochpass-Filter inverse Funktion übernimmt, können Daten gegebenenfalls blockiert werden.

Vorzugsweise umfassen die Daten Steuerdaten zur Steuerung des Moduls und/oder vom Modul bereitgestellte Messdaten.

Ein Modul kann typischerweise Funktionen übernehmen, welche mittels der Steuerdaten gesteuert werden können. Die Daten können mittels bekannter Protokolle codiert sein. Die Daten können auch Modulidentifikationen umfassen, welche das Modul identifizieren kann. Damit wird erreicht, dass die Steuerdaten jeweils von dem "richtigen" Modul verarbeitet, respektive in eine Funktion umgesetzt werden. Weiter ist auch denkbar, dass die Daten Zustandsinformationen des Moduls umfassen, dass mittels der Daten somit auch ein Zustand des Moduls geprüft wird, wobei der Zustand zum Beispiel als "aktiv", "nicht aktiv", "angeschlossen", "nicht angeschlossen", "defekt", "Betriebsdauer beträgt ...", "nächste Wartung fällig am ...", usw. bestimmt werden kann. Das Modul kann mittels der Daten auch Anweisungen erhalten, welche einen Gerätetest oder Anweisungen zum Ausführen eines Selbsttest beinhalten. Schliesslich können die Steuerdaten sogenannte Plug&Play ermöglichen, d.h. die Steuerdaten können so beschaffen sein, dass ein neues Modul automatisch erkannt und in Betrieb genommen wird ohne dass anschliessend irgendwelche zusätzlichen Massnahmen wie beispielsweise Treiber installiert oder andere Einstellungen vorgenommen werden müssen.

Die Daten können auch Messdaten umfassen, welche mittels eines bekannten Protokolls codiert sein und über die Versorgungsleitung übertragen werden können. Die Messdaten können von einem Modul generiert werden und mittels des Kopplers in die Versorgungsleitung eingekoppelt werden. Ein weiteres Modul kann mittels des Kopplers die Messdaten auskoppeln und zum Beispiel mittels einer RS-232-Schnittstelle einem PC zur Verfügung stellen. Der PC kann so programmiert sein, dass die Daten für die untersuchende Person automatisch als Bericht zusammengestellt werden. Damit wird erreicht, dass der Untersuchende kein handschriftliches Protokoll ausfüllen muss, womit bei den Untersuchungen Zeit und damit Kosten eingespart werden können.

Die Vorrichtung umfasst bevorzugt einen Stromanschluss zum Anschliessen an eine externe Energiequelle, wobei die Versorgungsleitung an den Stromanschluss angeschlossen, d. h. mit diesem verbunden ist. Alternativ könnte die Vorrichtung auch durch eine interne Energiequelle wie etwa eine Batterie gespiesen werden.

Vorzugsweise umfasst die Vorrichtung weiter ein Tiefpass-Filter, welches mit einer Schaltvorrichtung wahlweise in die Versorgungsleitung einfügbar bzw. aus dieser entfernbar ist. Der Tiefpass-Filter dient hierbei zur Filterung, d. h. Blockierung von über die Versorgungsleitung übertragenen Signalanteilen mit Frequenzen, die über der Grenzfrequenz des Tiefpass-Filters liegen. Hierzu gehören insbesondere die Signalanteile mit den über die Versorgungsleitung übertragenen Daten. Die Schaltvorrichtung kann hierbei insbesondere durch ein Modul steuerbar ausgebildet sein. Dadurch wird erreicht, dass bei eingeschaltetem Tiefpass-Filter die mittels der Versorgungsleitung übermittelten Daten nicht in die externe Energieversorgung eindringen können. Anderseits können die Daten bei ausgeschaltetem Tiefpass-Filter bewusst in die externe Energieversorgung gesendet werden. Diese können über eine weitere Vorrichtung aus der externen Energieversorgung ausgekoppelt werden. Die weitere Vorrichtung kann zum Beispiel auch als Vorrichtung zur Untersuchung und/oder Behandlung eines Auges, oder als PC ausgebildet sein.

Alternativ kann der Tiefpass-Filter auch permanent eingeschaltet sein, womit ein Missbrauch der Daten durch Dritte, welche Zugang zur externen Energieversorgung haben, respektive eine Störung von anderweitig über die externe Energieversorgung gesendeten Daten unterbunden wird. Schliesslich kann auf den Tiefpass-Filter auch verzichtet werden.

Typischerweise umfasst die Vorrichtung einen Transformator, der zwischen dem Stromanschluss und der mit dem Modul verbundenen Kommunikationsvorrichtung in die Versorgungsleitung geschaltet ist. Die Primärseite des Transformators ist in der Regel an eine (länderspezifische) Wechselstrom-Netzspannung angeschlossen, welche zum Beispiel in der Schweiz 230 V bei 50 Hz beträgt. Auch das Anschliessen der Vorrichtung an ein Drehstromnetz (z. B. 400 V , 50 Hz) ist denkbar. Anderseits dient die auf der Sekundärseite des Transformators abgreifbare Spannung zur Versorgung der Vorrichtung und der einzelnen Module mit elektrischer Energie, wobei der Transformator auch zur Bereitstellung von mehreren unterschiedlichen Sekundärspannungen ausgebildet sein kann, insbesondere wenn verschiedene Module vorhanden sind, welche unterschiedliche Spannungen aufnehmen. Der Transformator transformiert zum Beispiel einen eingehende Spannung (AC oder DC) auf 24V DC. Durch die Wahl einer tieferen Spannung wird erreicht, dass eine Verletzungsgefahr bei unsachgemässer Handhabung der Vorrichtung vermindert werden kann. Falls die Daten über die Netzleitung anderen Geräten (zum Beispiel einer weiteren Spaltlampe, einem PC, einem Server, etc.) zur Verfügung gestellt werden sollen, kann eine Vorrichtung vorgesehen sein, welche den Transformator für die Kommunikation transparent macht. Dies kann zum Beispiel durch einen entsprechenden Bypass für die Kommunikationssignale realisiert werden. Etwa in dem die Daten vor dem Transformator auskoppelt, einem Hochpassfilter zugeführt und hinter dem Transformator wieder einkoppelt werden.

Alternativ kann die Vorrichtung auch mit der Netzspannung betrieben werden, womit aber je nach deren Eigenschaften das Risiko einer Verletzung bei unsachgemässer Handhabung der Vorrichtung steigt. Dies hätte weiter den Nachteil, dass für jedes Gerät, welches eine andere Spannung benötigt, ein separater Transformator vorgesehen sein müsste, was einerseits teuer und anderseits platzaufwendig wäre. Stattdessen könnten auch Akkumulatoren verwendet werden, was dann von Vorteil ist, wenn die Vorrichtung in Bereichen verwendet werden soll, wo kein Netzstrom zur Verfügung steht, womit die Vorrichtung zum Beispiel als Feldgerät ausgebildet werden kann.

Bei einer weiteren Ausführungsvariante der Erfindung ist der Transformator als Teil des Moduls ausgebildet, d. h. das Modul umfasst den Transformator. Mindestens ein erstes Modul umfasst einen Transformator, wobei die andern Module beispielsweise vom ersten Modul mit elektrischer Energie versorgt werden. Durch das erste Modul wird damit die interne Versorgungsleitung gespiesen. Damit wird erreicht, dass genau ein Transformator für den Betrieb mehrerer Module ausreicht, womit ein konstruktiver Aufwand gering und damit die Herstellungskosten für die Vorrichtung tief gehalten werden können.

Der Transformator kann aber auch ausserhalb der Module angeordnet sein.

Vorzugsweise umfasst das Modul eine Beleuchtungsvorrichtung. Mittels der Kommunikationsvorrichtung kann das Modul Daten empfangen, welche zur Steuerung der Beleuchtungsvorrichtung verwendet werden können. Anderseits kann das die Beleuchtungsvorrichtung umfassende Modul mittels der Kommunikationsvorrichtung Daten senden, welche einen Zustand der Beleuchtungsvorrichtung wiedergeben. Dieser Zustand kann beispielsweise "an", "aus", "defekt", "nicht angeschlossen" sein. Weiter können die Daten Informationen über die aufgenommene Leistung oder der abgegebenen Lichtleistung der Beleuchtungsvorrichtung umfassen, welche relativ, als Prozentsatz der maximalen Leistung, oder absolut in Candela oder Watt übermittelt werden kann.

Das Modul kann auch andere steuerbare Vorrichtungen umfassen (siehe weiter unten).

Die Beleuchtungsvorrichtung umfasst bevorzugt zumindest eine LED. Die Verwendung von LED hat gegenüber Glühlampen gleich mehrere Vorteile:
1. Eine LED hat gegenüber Glühlampen eine wesentlich kürzere Ansprechzeit. Typischerweise liegen Ansprechzeiten einer LED im Bereich um 100 kHz. Damit können zum Beispiel Flicker-Frequenzen simuliert werden, womit das Auge auf Stresssituationen geprüft werden kann.
2. Eine LED emittiert ein weitgehend konstantes Farbspektrum, insbesondere kann damit beinahe monochromatisches Licht erzeugt werden. Durch die Wahl von LED's mit einem bestimmten, gewünschten Farbspektrum oder bei der Verwendung von LED's mit steuerbarem Farbspektrum (RGB) können Untersuchungen am Auge gezielt wellenlängenabhängig und reproduzierbar durchgeführt werden.
3. Das Farbspektrum einer LED ist weitgehend unabhängig von einer abgegebenen Lichtleistung der LED. Damit kann die Lichtleistung unabhängig einer Wellenlänge des emittierten Lichts erzeugt werden, womit wiederum die Reproduzierbarkeit der Messungen erhöht werden können.
4. Da die LED kein Temperaturstrahler ist, wird der Energieverbrauch vermindert. Weiter erhitzt sich das Gehäuse bedeutend weniger, womit Verbrennungen des Untersuchers sowie des zu Untersuchenden unterbunden werden können und die Material-beanspruchung der Untersuchungsvorrichtung verringert wird. Auch kann auf teure temperaturbeständige Gehäuseteile, insbesondere im Bereich der Lichtquelle, verzichtet werden, womit ein leichterer und kostengünstigerer Aufbau der Vorrichtung ermöglicht wird.
5. Die Lebensdauer einer LED ist wesentlich höher als diejenige von Glühlampen (Wolfram: 50 bis 300 Betriebstunden; LED: 30'000 bis 100'000 Betriebsstunden), womit zumindest längerfristig Kosten eingespart werden können. Weiter können durch den Einsatz einer LED Servicekosten gering gehalten werden, insbesondere, wenn die Lampen durch eine Fachperson ersetzt werden müssen.

Alternativ kann die Beleuchtungsvorrichtung auch eine oder mehrere Glühlampen umfassen. Diese haben jedoch den Nachteil, dass mit einer Einstellung der Helligkeit das Spektrum des erzeugten Lichtes nicht konstant gehalten werden kann. Dies könnte zwar mit Filter teilweise korrigiert werden, würde aber einen unverhältnismässigen Aufwand verursachen. Weiter ist auch der Einsatz von Halogen- oder Xenon-Lampen denkbar.

Die Beleuchtungsvorrichtung kann grundsätzlich zur Erzeugung jeder benötigten Art von Beleuchtung verwendet werden, beispielsweise für die Umfeldbeleuchtung oder auch für die Erzeugung des Fixierlichts. Vorzugsweise ist die Beleuchtungsvorrichtung allerdings zur Erzeugung einer Spaltbeleuchtung ausgebildet und verfügt hierfür über die entsprechenden Mittel wie Filter und Blenden etc. Auch diese Mittel können mittels der Steuerdaten angesteuert werden.

Für die Umfeldbeleuchtung umfasst die Beleuchtungsvorrichtung hingegen mit Vorteil einen Lichtleiter, der von der Beleuchtungsvorrichtung abzweigt. Damit wird eine besonders kompakte Bauform der Vorrichtung erreicht. Insbesondere können dadurch Stromleitungen eingespart und die Position der Umfeldbeleuchtung variabel eingestellt werden. Weiter kann auch eine Beleuchtungsvorrichtung zur Umfeldbeleuchtung vorgesehen sein. In einer bevorzugten Ausführungsform ist die Beleuchtungsvorrichtung gleichzeitig als Spaltbeleuchtungs-vorrichtung und als Umfeldbeleuchtungsvorrichtung ausgebildet. Dazu kann die Beleuchtungsvorrichtung ein oder mehrere LED's oder Glühlampen umfassen. Bevorzugt sind jedoch zwei Lichtquellen benachbart angeordnet, so dass eine kompakte Bauweise erreicht werden kann, bei welcher die Spaltbeleuchtung und die Umfeldbeleuchtung unabhängig voneinander angesteuert und eingestellt werden können. Die Lichtquellen sind dabei so angeordnet, dass der Spalt optimal beleuchtet werden kann. Das Licht für die Umfeldbeleuchtung wird über einen Lichtleiter in den Bereich des Umfelds geführt. Damit wird erreicht, dass die Umfeldbeleuchtung je nach Bedürfnis ausgerichtet werden kann. So kann zum Beispiel das distale Ende des Lichtleiters mit einer Vorrichtung verbunden sein, welche das Einstellen eines Winkels und/oder eine räumliche Anordnung des distalen Endes des Lichtleiters nach den momentanen Bedürfnissen erlaubt.

Die Umfeldbeleuchtung kann alternativ auch von einer externen Lichtquelle mittels eines Lichtleiters realisiert werden. Dies hat aber den Nachteil, dass längere Leiter verwendet und damit Lichtverluste und spektrale Farbverluste in Kauf genommen werden müssen, was nur teilweise durch eine leistungsstärkere Lichtquelle und damit mit einem erhöhten Energieverbrauch kompensiert werden könnte. Der konstruktive Aufwand würde sich damit auch erhöhen. Schliesslich wird dadurch die Mobilität der Vorrichtung beeinträchtigt.

Vorzugsweise umfasst das Modul ein steuerbares Element, welches beispielsweise ein elektrisches, ein elektronisches und/oder ein mechanisches Element sein kann. Beispielsweise ist eine LED der Beleuchtungsvorrichtung als elektrisches und/oder elektronisches steuerbares Element ausgebildet, wobei verschiedene Eigenschaften wie Helligkeit, Flickerfrequenz, Wellenlänge etc. steuerbar sind. Auch andere Elemente wie beispielsweise eine Datenverarbeitungseinrichtung eines Moduls oder auch die Kommunikationsvorrichtung selber können als steuerbares elektronisches Element ausgebildet sein.

Das Modul kann zum Beispiel eines der nachfolgenden steuerbaren mechanischen Elemente umfassen:
- Kreuzschlitten:: Das Modul umfasst mindestens ein Stellglied, welches insbesondere einen Schrittmotor oder einen Servomotor umfasst, mit welchem der Kreutzschlitten gesteuert werden kann. Bevorzugt umfasst der Kreutzschlitten mindestens zwei Stellglieder, so dass der Kreuzschlitten in einer Ebene bewegt werden kann, wobei ein erstes Stellglied eine geradlinige Bewegung des Kreuzschlittens erlaubt und ein zweites Stellglied eine geradlinige Bewegung rechtwinklig zu derjenigen des ersten Stellgliedes erlaubt. Statt eines Stellgliedes können auch andere Vorrichtungen zur Steuerung des Kreuzschlittens vorgesehen sein.
- Spalt:: Das Modul umfasst Mittel zur Dimensionierung des Spaltes für die Spaltbeleuchtung (Länge und Breite). Auch dazu umfasst das Modul ein oder zwei Stellglieder, je nach dem, ob eine Breite und/oder eine Höhe mittels des Moduls einstellbar ausgebildet sein soll.
- Farbfilter:: Bei der Beleuchtungsvorrichtung können Farbfilter eingesetzt werden, welche mittels eines steuerbaren mechanischen Elementes zugeschaltet oder gewechselt werden können. Farbfilter können auch im Strahlengang eines Mikroskops angeordnet und mittels des Moduls steuerbar ausgebildet sein.
- Imaging Modul:: Übertragungen von Bilddaten mittels einer Kamera können auch vorgesehen sein. Dabei kann zum Beispiel ein Fokkusieren oder ein Ausrichten der Kamera über ein steuerbares mechanisches Element erfolgen.

Diese Aufzählung ist nicht als abschliessend zu verstehen. Es ist zum Beispiel auch denkbar, mechanische Elemente von peripheren Geräten anzusteuern, wie Tonometer, Endothelmikroskop usw. Dies gilt natürlich auch für andere steuerbare elektrische oder elektronische Elemente.

Bei einer bevorzugten Ausführungsvariante umfasst das Modul eine Steuerung mit Bedienelementen. Diese dienen zur Bereitstellung von Steuerdaten, welche über die Versorgungsleitung übertragbar sind und mit welchem wenigsten ein steuerbares Element dieses oder wenigstens eines anderen Moduls gesteuert werden kann. Ein Modul mit einer solchen Steuerung wird nachfolgend auch als Bedien- oder Steuermodul bezeichnet. Ein solches Steuermodul kann insbesondere auch eine Anzeigevorrichtung wie beispielsweise ein Display umfassen. Damit wird eine übersichtliche Bedienung der Vorrichtung erreicht, insbesondere wenn die Steuerung mehrerer Module über das Bedienmodul/Steuermodul vorgenommen werden kann. Die Bedienelemente können als Schalter oder Knöpfe für die Steuerung von Funktionen eines Moduls ausgebildet sein, wie zum Beispiel das Ein- und Ausschalten von Beleuchtungsvorrichtungen. Weiter können auch Drehknöpfe vorgesehen sein, um zum Beispiel die Helligkeit der Beleuchtungsvorrichtungen einstellen zu können. Die Bedienelemente können zum Beispiel als Potentiometer oder als Inkrementalgeber ausgebildet sein. Auch weitere, dem Fachmann in einer Vielzahl bekannte Bedienelemente können eingesetzt werden. Auf dem Display können zum Beispiel das Modul oder mehrere Module mittels eines Bedienelements anwählbar und steuerbar sein. Mittels des Displays können auch vom Modul gesendete oder empfangene Daten dargestellt werden. So können zum Beispiel Messdaten, Zustand des Moduls oder eine Liste der vorhandenen Module angezeigt werden. Als Zustand des Moduls kann über das Display die momentane Einstellung der Funktion des Moduls, respektive "on" oder "off" und dergleichen ausgegeben werden. Das Display kann als Touchscreen ausgebildet sein, so dass es die Funktionen eines Bedienmoduls/Steuermoduls übernehmen kann und damit die Bedienelemente beinhaltet. Falls die Vorrichtung eine Kamera umfasst, können auch Bild oder Videodaten auf dem Display dargestellt werden. Die Module können auch jeweils über ein separates Bedienmodul/Steuermodul steuerbar ausgebildet sein, womit eine individuelle Positionierung der Bedienmodule/Steuermodule ermöglicht wird. Damit würde aber der Aufbau der Vorrichtung aufwendiger und damit auch teuerer werden.

Bevorzugt umfasst die Vorrichtung, welche beispielsweise als Spaltlampenmikroskop ausgebildet ist, einen in mehreren Raumrichtungen bewegbaren Schlitten und einen auf dem Schlitten angeordnetes Mikroskop, wobei das Steuermodul an dem Schlitten angeordnet ist. Damit wird eine für den Untersuchenden besonders ergonomische Anordnung der Bedienelemente des Steuermoduls erreicht, da die untersuchende Person mit minimalen Bewegungen zwischen den Einstellungen am Kreutzschlitten und den Einstellungen am Steuermodul hantieren kann und somit seinen Blick nicht vom Mikroskop lösen muss.

Die Bedienelemente oder das ganze Steuermodul können auch als Fernbedienung ausgebildet und lösbar am Schlitten angeordnet sein, so dass die Bedienelemente oder das Steuermodul auch an einem beliebigen weiteren Ort, an oder im Bereich der Vorrichtung untergebracht werden kann. Die untersuchende Person erhält dadurch die Freiheit, die Bedienelemente nach seinen Bedürfnissen und Vorzügen zu platzieren. Dabei können die Bedienelemente oder das Steuermodul via Kabel oder auch Funk angebunden sein.

Bevorzugt umfasst das Modul eine Speichereinheit und eine Recheneinheit, wobei in der Speichereinheit ein Programm speicherbar ist und wobei das Programm von der Recheneinheit ausführbar und mittels Daten, die über die Versorgungsleitung empfangen worden sind, steuerbar ist. Damit wird erreicht, dass im Modul selber ein Programm ausführbar ist, das von aussen angestossen werden kann. D. h. mittels relativ geringer Bandbreiten für die Datenübertragung zum Modul können trotzdem komplexere Steuerungen vorgenommen werden. Die Programme können in der Speichereinheit fest gespeichert sein oder über eine Schnittstelle, vorzugsweise eine RS-232-Schnittstelle, zum Modul übertragen und in die Speichereinheit eingespeichert werden, so dass eine Aktualisierung oder eine Änderung der Steuerung in einfacher Weise vorgenommen werden kann.

Bei einfacheren Steuerungen kann auf eine Speichereinheit und eine Recheneinheit auch verzichtet werden.

Aus der nachfolgenden Detailbeschreibung und der Gesamtheit der Patentansprüche ergeben sich weitere vorteilhafte Ausführungsformen und Merkmalskombinationen der Erfindung.

### Kurze Beschreibung der Zeichnungen

Die zur Erläuterung des Ausführungsbeispiels verwendeten Zeichnungen zeigen:
- Fig. 1: eine vereinfachte schematische Darstellung der Vorrichtung zur Untersuchung und/oder Behandlung eines Auges;
- Fig. 2: eine Schrägansicht eines Spaltlampenmikroskops mit einer zu untersuchenden Person;
- Fig. 3: eine schematische Darstellung der Vorrichtung zur Untersuchung und/oder Behandlung eines Auges;
- Fig. 4: ein erfindungsgemässer Systemaufbau über das Stromnetz einer Hausinstallation;

Grundsätzlich sind in den Figuren gleiche Teile mit gleichen Bezugszeichen versehen.

### Wege zur Ausführung der Erfindung

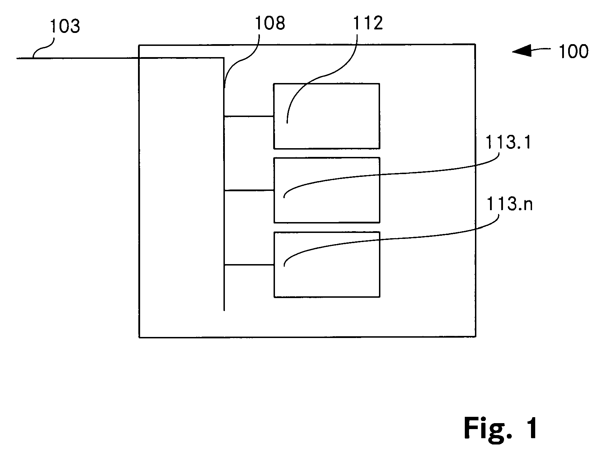

In der **Figur 1** ist ein einfacher schematischer Aufbau einer möglichen Ausführungsform dargestellt. Ein lokales Subnetz 100 ist als Spaltlampenmikroskop 100 ausgebildet. Das Spaltlampenmikroskop 100 umfasst eine Verbindungsleitung 103 zu einer Stromversorgungseinrichtung (nicht dargestellt). Weiter umfasst das Spaltlampenmikroskop eine lokale (interne) Versorgungsleitung 108. Mehrere Module 112, 113.1, 113.n sind mit der lokalen Versorgungsleitung 108 verbunden. Das Modul 112 ist als Steuermodul mit wenigstens einem Bedienelement ausgebildet, das Modul 113.1 umfasst die Spaltbeleuchtung und die Umfeldbeleuchtung und das Modul 113.n kann zum Beispiel eine weitere Umfeldbeleuchtung umfassen. Natürlich können auch mehr als die drei in der **Figur 1** dargestellten Module vorhanden sein. Zwischen der Verbindungsleitung 103 und der lokalen Versorgungsleitung 108 kann ein Transformator angeordnet sein.

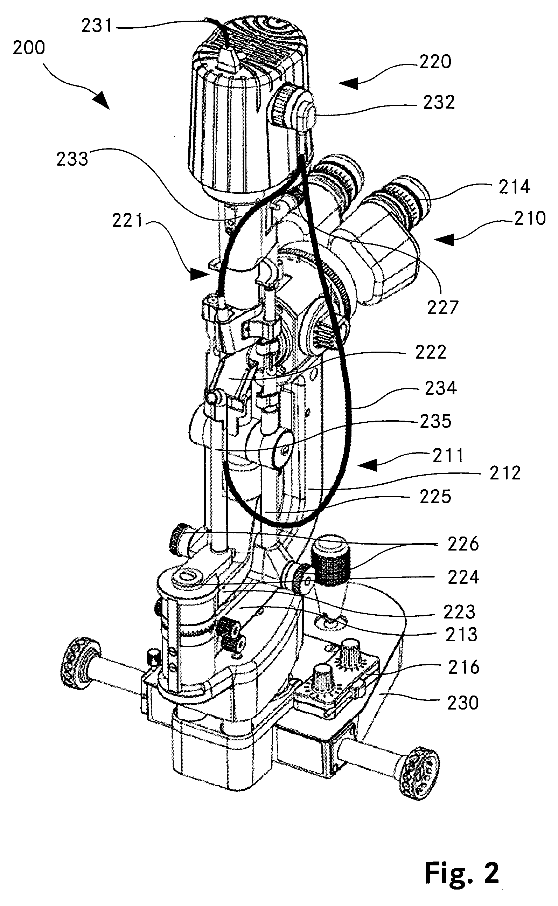

Das in der **Figur 2** dargestellte sogenannte Spaltlampenmikroskop (wird auch Spaltlampe genannt) als eine mögliche Realisierung eines Subnetzes 200 als Vorrichtung zur stereoskopischen Betrachtung eines Auges hat eine Betrachtungseinheit 210 und eine Beleuchtungseinheit 220. Die Betrachtungseinheit 210 ist mit einer Halteeinheit 211 und die Beleuchtungseinheit 220 mit einer Halteeinheit 221 gehalten. Mit der Beleuchtungseinheit 220 ist ein schmaler Lichtstreifen als Strahlung (Spalt) erzeugbar, der über einen an der Halteeinheit 221 angeordneten Umlenkspiegel 222 in bzw. auf das Auge führbar ist. Die Halteeinheit 221 ist über ein Schwenkgelenk 224 mit einer vertikalen Schwenkachse verschwenkbar an einem Vorrichtungsfuss 230 angeordnet.

Die, Halteeinheit 221 ist als L-förmiges Bauteil ausgebildet, welches am Ende eines horizontal verlaufenden Schenkels 223 in einem um eine vertikale Achse verschwenkbaren Schwenkgelenk 224 verschwenkbar am Vorrichtungsfuss 230 angeordnet ist. Der andere Schenkel 225 der Halteeinheit 221 verläuft vertikal. Innen ist der Schenkel 225 hohl. In seinem Hohlraum verläuft ein (nicht dargestellter) stangenförmiger Einstellmechanismus zur Einstellung einer Spaltbreite in der Beleuchtungseinheit 220, welche am oberen Ende des Schenkels 225 angeordnet ist. Eine Einstellung der Spaltbreite erfolgt über die Einstellknöpfe 226, welche im Übergansbereich der Schenkel 223, 225 angeordnet sind.

Die Betrachtungseinheit 210 ist analog zur Beleuchtungseinheit 220 ebenfalls auf einer L-förmigen Halteeinheit 211 angeordnet. Auch diese Halteeinheit 211 hat einen horizontal und einen vertikal liegenden Schenkel 212 und 213. Das Ende des horizontalen Schenkels 213 ist analog zur Halteeinheit 221 auch um dessen vertikale Achse verschwenkbar und mit dem nach unten verlängerten Schwenkgelenk 224 am Vorrichtungsfuss 230 unabhängig gegenüber der Halteeinheit 221 verschwenkbar gelagert. Am oberen Ende des Schenkels 212 ist die Betrachtungseinheit 210 in einer Höhe angeordnet, welche einen Blick in das Auge erlaubt.

In einem Strahlengang kann eine Bildauskopplung für eine Aufnahmeeinheit vorgesehen sein. Diese kann mit einem Teilerprisma, welches den Strahlengang in einen Teilstrahl zum Okular 214 sowie in einen weiteren Teilstrahl über ein Video-Objektiv zu einer Aufnahmeeinheit aufteilt (nicht dargestellt). Neben einer visuellen Betrachtung können somit zusätzlich Videoaufnahmen zur direkten Betrachtung oder zur Aufzeichnung (Dokumentation) vorgenommen werden (nicht dargestellt).

Mittels eines in der Beleuchtungseinheit 220 angeordneten Verstellhebels 227 sind die Höhe des auf das Auge zu richtenden Lichtspaltes sowie das Zuschalten eines Blaufilters wählbar. Mit einem im Bereich des Verstellhebels 227 angeordneten Verstellhebels (nicht dargestellt) können nicht explizit dargestellte weitere Farbfilter und andere optische Elemente in den Beleuchtungsstrahlengang gebracht werden.

Die Beleuchtungseinheit 220 umfasst einen zweipoligen Anschluss 231, über welchen sowohl elektrische Energie wie auch Daten übermittelbar sind. Innerhalb der Beleuchtungseinheit 220 ist ein Modul angeordnet, welches eine PLC (powerline communication)-Einheit 110, eine Funktionseinheit 111 mit Prozessor umfasst, welche anhand empfangener Daten LED's für die Beleuchtungsvorrichtung 111.1 steuern kann (nicht ersichtlich). Für die Umfeldbeleuchtung und die Spaltbeleuchtung 111.1 sind separate LED's vorgesehen. Die Steuerung der Beleuchtungsvorrichtung 111.1 (Spaltbeleuchtung und Umfeldbeleuchtung) erfolgt über die Bedieneinheit 216, welche dazu zwei Drehknöpfe umfasst, mittels welcher die Intensität der Beleuchtungen stufenlos eingestellt werden kann. Die mit dem Bedienelement 216 erzeugten Steuerdaten werden mittels einer PLC-Einheit 110 (nicht sichtbar) in die Versorgungsleitung eingekoppelt, über die Versorgungsleitung zur Beleuchtungsvorrichtung 111.1 gesendet und von einer PLC-Einheit 110 der Beleuchtungsvorrichtung 111.1 ausgekoppelt. Die Steuerung der Intensitäten der LED's 111.4 in der Beleuchtungsvorrichtungen 111.1 erfolgt anschliessend aufgrund der gesendeten Steuerdaten. Damit die Beleuchtungseinheiten einzeln über dieselbe Versorgungsleitung angesteuert werden können, werden mit den Steuersignalen nicht nur die Einstelldaten für die Beleuchtung, sondern auch eine Adresse mit gesendet, so dass jeweils die "richtige" der beiden Leuchtquellen angesteuert wird. Am Faseraustritt 232 können Lichtleiter 233 und 234 für die Umfeldbeleuchtung angeschlossen werden. Der Lichtleiter 233 wird oberhalb des Umlenkspiegels 222 angeschlossen, so dass das aus dem Lichtleiter 233 austretende Licht auf den Umlenkspiegel auftrifft. Der Lichtleiter 234 für die Umfeldbeleuchtung ist auf einer L-förmigen Halteeinheit 235 angeordnet. Auch diese Halteeinheit 235 ist analog zur Halteeinheit 221 auch um dessen vertikale Achse verschwenkbar und am Vorrichtungsfuss 230 unabhängig gegenüber der Halteeinheit 221 verschwenkbar gelagert. Am oberen Ende eines vertikalen Schenkels ist das Ende des Lichtleiters 234 für die Umfeldbeleuchtung befestigt.

In einer weiteren Ausführungsform können die Lichtleiter 233, 234 auch innerhalb eines Gehäuses verlaufen, um das Spaltlampenmikroskop 100 optisch ansprechender gestalten zu können. Die Spaltbeleuchtungsvorrichtung und die Umfeldbeleuchtungsvorrichtung können auch in zwei separaten Modulen vorgesehen sein. In einem Modul können aber auch mehr als zwei Funktionen untergebracht werden. Gegebenenfalls kann der Faseraustritt 232 auch nur einen Anschluss für einen Lichtleiter aufweisen, womit die Vorrichtung entweder die Umfeldbeleuchtung 233 oder die Umfeldbeleuchtung 234 umfasst.

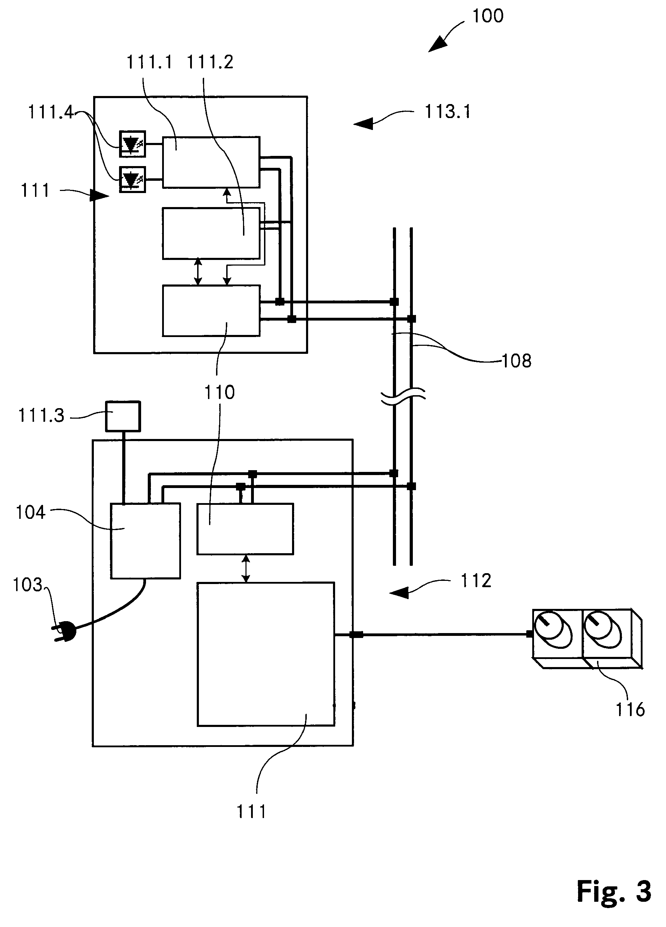

Die **Figur 3** zeigt einen detaillierteren schematischen Aufbau eines Spaltlampenmikroskops 100. Das Spaltlampenmikroskop 100 umfasst eine lokale Versorgungsleitung 108, an welche in der vorliegenden Ausführungsform zwei Module 112, 113.1 angeschlossen sind. Ein erstes Modul 112 umfasst eine Verbindungsleitung 103 zu einer Stromversorgungseinrichtung (nicht dargestellt). Ein Transformator 104 ist über einen ersten Anschluss mit der Verbindungsleitung 103 verbunden und mit einem zweiten Anschluss mit der lokalen Versorgungsleitung 108 verbunden. Eine Fixierlampe 111.3 ist direkt mit dem Transformator 104 verbunden. Weiter umfasst das Modul 112 eine mit der lokalen Versorgungsleitung 108 verbundene PLC-Einheit 110, welche in Kommunikation mit einer Funktionseinheit 111 steht. Die Funktionseinheit 111 umfasst ein Bedienelement 116. Das Bedienelement 116 ist als Potentiometer mit zwei skalierten Drehknöpfen ausgebildet.

Das zweite Modul 113.1 ist über eine PLC-Einheit 110 mit der lokalen Versorgungsleitung 108 verbunden. Das Modul 113.1 umfasst eine Funktionseinheit 111 mit Prozessor, welche eine Beleuchtungsvorrichtung 111.1 (Spalt- und Umfeldbeleuchtung) mit Dioden 111.4 und eine weitere Vorrichtung 111.2 umfasst. Diese sind auch an die lokale Versorgungsleitung 108 angeschlossen und über die PLC-Einheit 110 steuerbar. Die weitere Vorrichtung 111.2 kann dabei eine weitere Beleuchtung, die Einstellung einer Spaltbreite und/oder Spalthöhe, eine Kamera, einen zuschaltbaren Farbfilter oder weitere steuerbare und/oder messbare Funktionen umfassen.

Die lokale Versorgungsleitung ist zweipolig ausgeführt und mittels des Transformators 104 mit 24 V (Volt) und mindestens 3 A (Ampere) Gleichstrom (DC) oder Wechselstrom (AC) beaufschlagt. Der Transformator selbst wird über einen üblichen, länderspezifischen Stromanschluss gespiesen, insbesondere mit 230V Wechselstrom. Die PLC-Einheit 110 separiert den elektrischen Strom zur Energieversorgung und die gesendeten Daten.

Mit dem Bedienelement 116 können nun Einstellungen der Intensität und/oder Frequenz der Dioden 111.4 vorgenommen werden, wobei der eine Drehknopf des Bedienelement 116 zur Steuerung der Dioden 111.4 der Spaltbeleuchtungsvorrichtung, insbesondere einer Intensität der Dioden 111.4 der Spaltbeleuchtungsvorrichtung und der zweite Drehknopf des Bedienelements 116 zur Steuerung der Dioden 111.4 der Umfeldbeleuchtungsvorrichtung, insbesondere einer Intensität der Dioden 111.4 der Umfeldbeleuchtungsvorrichtung vorgesehen ist. Die von der Funktionseinheit 111 des ersten Moduls 112 erfassten Daten werden mittels PLC-Einheit 110 des ersten Moduls 112 in die lokale Versorgungsleitung 108 eingekoppelt und versendet. Die PLC-Einheit 110 des zweiten Moduls 113.1 empfängt die Daten und koppelt sie aus der lokalen Versorgungsleitung 108. Daten werden in Abhängigkeit einer Adresse eines Protokolls (siehe unten) mittels einer Prozessoreinheit der PLC-Einheit 110 entweder an die Spaltbeleuchtungsvorrichtung oder an die Umfeldbeleuchtungsvorrichtung gesendet. Die Intensitäten der Beleuchtungen werden den empfangenen Daten entnommen.

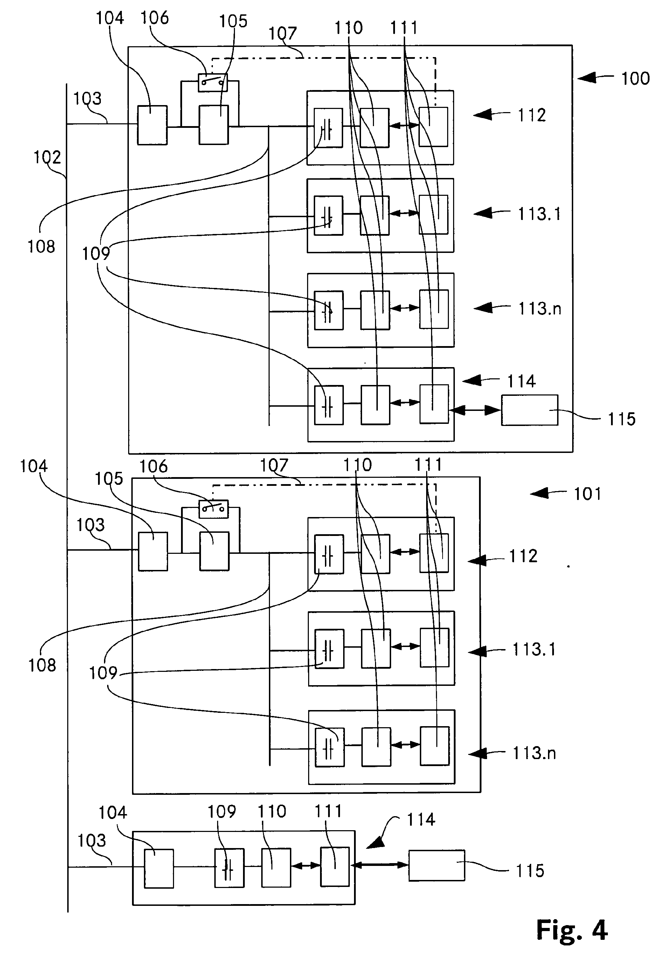

In der **Figur 4** ist ein Aufbau, respektive eine Topologie eines Systemaufbaus dargestellt, bei welchem die Kommunikation zwischen zwei oder mehr Modulen 112, 113.1, 113.n nicht nur lokal in einer nach aussen isolierten Zelle, insbesondere in einem Spaltlampenmikroskop stattfindet, sondern auch global über die (hauseigene) Versorgungsleitung 102 erfolgen kann. In der vorliegenden Ausführungsform ist die Versorgungsleitung mit 230V AC beaufschlagt, was aber je nach Land verschieden gewählt werden kann. Die Einkopplung und Auskopplung von Daten in und aus einer Versorgungsleitung 102, 108, erfolgt durch einen Router 114 nach dem FSK-Verfahren (Frequency Shift Keying), wobei auch hier andere, dem diesbezüglichen Fachmann bekannte Verfahren verwendet werden können. Die Daten können Steuersignale, Messdaten, Identifikationsdaten, Prüfdaten, etc. umfassen.

Das gesamte System besteht aus mehreren nach aussen isolierten Subnetzen 100, 101, wobei in der **Figur 4** exemplarisch zwei Subnetze 100, 101 dargestellt sind, nämlich ein erstes Subnetz 100 und ein zweites Subnetz 101. Der Router 114 ist verbunden mit einer PC-Schnittstelle 115. Die Subnetze 100, 101 umfassen jeweils ein Netzteil 104, ein überbrückbarer Tiefpass-Filter 105, eine lokale Versorgungsleitung 108 und ein oder mehrere Module 112, 113.1, 113.n. Ein lokales Subnetz ist dabei als Vorrichtung zur Untersuchung und/oder Behandlung eines Auges, insbesondere als Spaltlampenmikroskop ausgebildet. Auch weitere Subnetze 100, 101 können als Vorrichtung zur Untersuchung und/oder Behandlung eines Auges oder realisiert sein. So können zum Beispiel in einem Spital mehrere solche Vorrichtungen bezüglich eines Datenflusses gekoppelt und die gemessenen Daten auf einem gemeinsamen Server gespeichert oder weiter verarbeitet werden.

Die Subnetze 100, 101 sind jeweils mit einem ersten Anschluss eines Netzteils 104 über eine Verbindungsleitung 103 mit der Versorgungsleitung 102 verbunden, so dass die Subnetze 100, 101 mit elektrischer Energie versorgt werden können, welche die gewünschte Spannung aufweist. In der vorliegenden Ausführungsform beträgt die Spannung an einem zweiten Anschluss des Netzteils 104 24V DC, wobei auch andere Spannungen und Spannungsformen wie AC-Spannungen denkbar sind. Das Netzteil 104 ist mit seinem zweiten Anschluss mit einem ersten Anschluss eines Tiefpass-Filters (TP-Filter) 105 verbunden, welcher mittels eines Umschalters 106 überbrückbar ausgebildet ist (siehe unten). Ein zweiter Anschluss des TP-Filters 105 ist mit der lokalen Versorgungsleitung 108 verbunden. Mit der lokalen Versorgungsleitung 108 sind ein oder mehrere Module 112, 113.1, 113.n, sowie (bei mindestens einem Subnetz) ein Router 114 mit einer PC-Schnittstelle verbunden. Das Netzteil 104 ist dabei bevorzugt mindestens im Frequenzbereich der zu übertragenden Daten transparent, d. h. das Netzteil ist bevorzugt so beschaffen, dass die Daten vor und nach dem Netzteil 104 interpretierbar, respektive identisch bleiben. Dies kann dadurch erreicht werden, dass die Daten um das Netzteil 104 geleitet werden. Vor dem Netzteil 104 können die Daten durch einen Hochpassfilter ausgekoppelt werden und nach dem Netzteil 104 wieder eingekoppelt werden (nicht dargestellt).

Ein Modul 112, 113.1, 113.n kann entweder als Master-Modul 112 oder als Slave-Modul 113.1, 113.n ausgebildet sein. Ein Subnetz 100, 101 umfasst mindestens ein Master-Modul 112 und kann ein oder mehrere Slave-Module 113.1, 113.n umfassen. Ein Modul 112, 113.1, 113.n umfasst eine PLC-Einheit 110 mit einem Hochpass-Filter 109 zum einrespektive auskoppeln von Daten in, respektive aus der lokalen Versorgungsleitung 108 und eine Funktionseinheit 111. Der Hochpass-Filter 109 ermöglicht dabei ein Abzweigen von Daten enthaltenden hochfrequenten Signalen. Die Funktionseinheit umfasst typischerweise einen Prozessor, eine Speichereinheit für eine Software sowie einen funktionellen Teil, welcher mittels der Software gesteuert, geprüft, ein- und ausgeschalten, identifiziert etc. werden kann. Der funktionelle Teil umfasst eine steuerbare Funktion, eine steuernde Funktion oder einen Messwertgeber. Der funktionelle Teil kann eine Bedieneinheit, eine Beleuchtungseinheit, ein Tonometer, einen Kreuzschlitten, einen Farbfilter, eine Kamera oder ähnliches umfassen. Die von den Modulen 112, 113.1, 113.n benötigte Energie beziehen diese von der lokalen Versorgungsleitung 108.

Die Module 112, 113.1, 113.n und der Router 114, respektive der am Router 114 angeschlossene PC können über die gemeinsame Versorgungsleitung 108 untereinander Daten austauschen. Die Daten können wahlweise in der globalen Versorgungsleitung zur Verfügung gestellt werden, indem der Tiefpass-Filter 105 mittels des geschlossenen Umschalters 106 überbrückt wird, oder nicht zur Verfügung gestellt werden, in dem der Tiefpass-Filter 105 mittels des offenen Umschalters 106 nicht überbrückt wird. Die Steuerung des Umschalters 106 erfolgt in der vorliegenden Ausführungsform durch den Prozessor, respektive die Anwendungssoftware des Master-Moduls 112.

Bei lokalem Betrieb ist eine Kommunikation zwischen den Modulen 112, 113.1, 113.n nach aussen, d.h. in der globalen Versorgungsleitung 102 nicht sichtbar, da der TP-Filter 105 die auf die lokale 24V Versorgungsleitung 108 aufmodulierten, respektive eingekoppelten Daten nicht auf die Versorgungsleitung 102 gelangen lässt. In diesem Modus ist der Umschalter 106 offen, so dass der TP-Filter 105 wirken kann.

Bei globalem Betrieb ist der Umschalter 106 geschlossen, so dass der TP-Filter 105 überbrückt ist, womit die Signale über die Versorgungsleitung 102 auch zwischen den lokalen Subnetzen 100, 101 ausgetauscht werden können. Die Kommunikation erfolgt dann zum Beispiel gemäss CENELEC Normen über die Hausinstallation.

Ein TP-Filter erlaubt Signalanteilen mit Frequenzen unterhalb einer Grenzfrequenz zu passieren, während Signalanteile mit Frequenzen über der Grenzfrequenz abgeschwächt oder vollständig blockiert werden. Damit können gewissermassen die mittels der Versorgungsleitung übertragenen Daten durch den TP-Filter gelöscht werden.

Mindestens ein lokales Subnetz 100, 101 umfasst einen Router 114 mit einer PC-Schnittstelle 115. Weiter ist ein Router 114 mit der globalen Versorgungsleitung 102 und mit einer PC-Schnittstelle 115 verbunden. Der Router 114 umfasst eine PLC-Einheit 110 zum ein- respektive auskoppeln von Daten in respektive aus der lokalen Versorgungsleitung 108 und eine Funktionseinheit 111. Die Funktionseinheit 111 umfasst wiederum einen Prozessor, eine Speichereinheit, eine Anwendungssoftware, sowie eine Schnittstelle für einen PC. Die Schnittstelle ist in der vorliegenden Ausführungsform als RS-232-Schnittstelle ausgebildet und ermöglicht einen Datenaustausch zwischen dem Router und dem PC. Natürlich können auch andere dem Fachmann bekannte Schnittstellen eingesetzt werden, wie zum Beispiel USB, Bluetooth etc.. Der Router 114 entkoppelt (demoduliert) die über die Versorgungsleitung 102, 108 gesendeten Daten mittels FSK-Verfahren und sendet diese an eine Standard RS-232 Schnittstelle. An der Schnittstelle kann ein beliebiges Kommunikationsgerät angeschlossen werden, welches mit der RS-232-Schnittstelle kompatibel ausbildbar ist (insbesondere auch über Adapter). In der vorliegenden Ausführungsform ist das Kommunikationsgerät als PC ausgebildet. Umgekehrt kann der PC, oder ein anderes mit RS-232 kompatibles Gerät, Daten an den Router 114 senden, wobei der Router die Daten mittels FSK-Verfahren in die Versorgungsleitung 102, 108 einkoppelt (aufmoduliert). Ein Router 114 kann sowohl direkt mit der globalen Versorgungsleitung 102 wie auch mit der lokalen Versorgungsleitung 108 verbunden sein. In der vorliegenden Ausführungsform ist sowohl ein Router 114 mit der globalen Versorgungsleitung 102 wie auch ein Router 114 mit der lokalen Versorgungsleitung 108 verbunden.

Die zwischen den Modulen 112, 113.1, 113.n respektive den Subnetzen 100, 101 und den Router 114 ausgetauschten Daten werden als CR-terminierte ASCII-Strings übertragen. Dazu werden binäre Daten durch den Sender in lesbare ASCII-Zeichen kodiert, wobei das Abschluss-Zeichen immer ein ASCII-CR-Charakter ist. Jeder Datenstring enthält weiter eine Längs-Checksumme, mit welcher Übertragungsfehler erkannt werden können.

Das Master-Modul 112 spricht ein gewünschtes Slave-Modul 113.1, 113.n mit seiner Adresse an. Der Slave übernimmt die Daten und führt das an ihn gerichtete Kommando aus. Abhängig von der Art des Kommandos gibt das Slave-Modul Daten an das Master-Modul zurück. Werden Daten zurückgegeben, werden diese innerhalb einer definierten Antwort-Zeit erwartet. Bei Überschreitung der Antwort-Zeit registriert das Master-Modul ein Fehlerereignis.

Die Daten werden gemäss einem Protokoll übertragen, welches folgendes umfasst: Adresse; Befehle; Daten; Checksumme; Abschluss-Zeichen.

Die Adresse ermöglicht eine eindeutige Zuweisung des Datenstrings an ein Modul.

Die Befehle können beispielsweise folgendes umfassen:
1. Anweisung an das Modul, seine Identifikation zu senden.
2. Anweisung an das Modul, seine Herstellungsdaten zu senden.
3. Anweisung an das Modul, bei der Funktionseinheit 111 Einstellungen vorzunehmen (Helligkeit von Umfeld und/oder Spaltbeleuchtung; Dimension des Spalts; Position des Kreutzschlittens etc.).
4. Anweisung an das Modul, in den Standby-Modus zu gehen.
5. Anweisung an die Module, mittels eines Resets mit Standardwerten neu zu starten.
6. Anweisung an das Mastermodul, Konfigurationsdaten zu senden.

Diese Liste ist nicht abschliessend zu verstehen und kann beliebig erweitert werden. Das obig beschriebene Protokoll ist lediglich als exemplarische Ausführung zu verstehen. Dem Fachmann ist klar, dass beliebige andere Protokolle für diese Anwendung geeignet sind.

In einer weiteren Ausführungsform kann das System auch nur eine Versorgungsleitung 102 und ein Subnetz 100 umfassen, wobei ein Datenaustausch nur lokal erfolgt.

Zusammenfassend ist festzustellen, dass durch die Erfindung eine Vorrichtung zur Untersuchung und/oder Behandlung eines Auges geschaffen wird, bei welcher mittels einer Versorgungsleitung Module mit elektrischer Energie versorgt werden können und zugleich über dieselbe Versorgungsleitung Daten zwischen den Modulen übertragbar sind, welche insbesondere als Steuerdaten, Messdaten, Modultestdaten und Modulidentifikationsdaten ausgebildet sind.

## Patentansprüche

1. Vorrichtung zur Untersuchung und/oder Behandlung eines Auges (100) mit einem in mehreren Raumrichtungen bewegbaren Schlitten, einem auf dem Schlitten angeordneten Mikroskop und einer Versorgungsleitung (102) und einem Modul (112, 113.1, 113.2. 113.3, 113.n), welches zur Versorgung mit elektrischer Energie mit der Versorgungsleitung (102) verbunden ist, wobei das Modul (112, 113.1, 113.2, 113.3, 113.n) mit einer an die Versorgungsleitung (102) angeschlossenen Kommunikationsvorrichtung (110) zum Senden und/oder Empfangen von Daten über die Versorgungsleitung (102) verbunden ist, ein steuerbares elektrisches, elektronisches und/oder mechanisches Element und eine Steuerung mit Bedienelementen (116; 216) zur Bereitstellung von über die Versorgungsleitung (102) übertragbaren Steuerdaten umfasst, **dadurch gekennzeichnet, dass** das Modul (112, 113.1, 113.2, 113.3, 113.n) an dem Schlitten angeordnet ist.

2. Vorrichtung nach Anspruch 1, **dadurch gekennzeichnet, dass** die Daten in Form eines elektrischen Signals mit einer Frequenz grösser als 100 Hz über die Versorgungsleitung (102) übertragbar sind, wobei die Frequenz insbesondere höher als 1 kHz, vorzugsweise im Bereich von 50 kHz bis 2 MHz ist,

3. Vorrichtung nach einem der Ansprüche 1 bis 2, **dadurch gekennzeichnet, dass** die Kommunikationsvorrichtung einen Koppler umfasst, mit welchem Daten in die Versorgungsleitung (102) ein- und/oder aus der Versorgungsleitung (102) auskoppelbar sind,

4. Vorrichtung nach einem der Ansprüche 1 bis 3, **dadurch gekennzeichnet, dass** die Daten mittels Frequenzumtastung über die Versorgungsleitung (102) übertragbar sind.

5. Vorrichtung nach einem der Ansprüche 1 bis 4, **dadurch gekennzeichnet, dass** die Daten Steuerdaten zur Steuerung des Moduls (112, 113.1, 113.2, 113.3, 113.n) und/oder vom Modul (112, 113.1, 113.2, 113.3, 113.n) bereitgestellte Messdaten umfassen.

6. Vorrichtung nach einem der Ansprüche 1 bis 5, **dadurch gekennzeichnet, dass** sie zum Anschliessen an eine externe Energiequelle einen Stromanschluss umfasst, mit welchem die Versorgungsleitung (102) verbunden ist, und die Vorrichtung vorzugsweise ein Tiefpass-Filter (105) umfasst, welcher mit einer Schaltvorrichtung (106) wahlweise in die Versorgungsleitung (102) einfügbar bzw. aus dieser entfernbar ist.

7. Vorrichtung nach Anspruch 6, **dadurch gekennzeichnet, dass** sie einen Transformator (104) umfasst, der zwischen dem Stromanschluss und der Kommunikationsvorrichtung (110) in die Versorgungsleitung (102) geschaltet ist.

8. Vorrichtung nach Anspruch 7, **dadurch gekennzeichnet, dass** das Modul (112, 113.1, 113.2, 113.3, 113.n) den Transformator (104) umfasst.

9. Vorrichtung nach einem der Ansprüche 1-8, **dadurch gekennzeichnet, dass** das Modul (112, 113.1, 113.2, 113.3, 113.n) eine Beleuchtungsvorrichtung (111.1 - 111.4) umfasst.

10. Vorrichtung nach Anspruch 9, **dadurch gekennzeichnet, dass** die Beleuchtungsvorrichtung (111.1 - 111.4) zumindest eine LED (111.4) umfasst und insbesondere zur Erzeugung einer Spaltbeleuchtung (111.1) ausgebildet ist.

11. Vorrichtung nach einem der Ansprüche 9 bis 10, **dadurch gekennzeichnet, dass** sie einen von der Beleuchtungsvorrichtung (111.1 - 111.4) abzweigenden Lichtleiter (234) für eine Umfeldbeleuchtung umfasst.

12. Vorrichtung nach einem der Ansprüche 1 bis 11, **dadurch gekennzeichnet, dass** das Modul (112, 113.1, 113.2, 113.3, 113.n) eine Anzeigevorrichtung umfasst.

13. Vorrichtung nach einem der Ansprüche 1 bis 12, **dadurch gekennzeichnet, dass** das Modul (112, 113.1, 113.2, 113.3, 113.n) eine Speichereinheit und eine Recheneinheit (111) umfasst, wobei in der Speichereinheit ein Programm speicherbar und das Programm von der Recheneinheit ausführbar und mittels über die Versorgungsleitung (102) empfangenen Daten steuerbar ist.

## Claims

1. Device for examining and/or treating an eye (100), having a slide which can be moved in a plurality of spatial directions, a microscope arranged on the slide and a supply line (102) and a module (112, 113.1, 113.2, 113.3, 113.n) which is connected to the supply line (102) in order to be supplied with electrical energy, the module (112, 113.1, 113.2, 113.3, 113.n) being connected to a communication device (110), which is connected to the supply line (102) and is intended to transmit and/or receive data via the supply line (102), and comprising a controllable electrical, electronic and/or mechanical element and a controller with operating elements (116; 216) for providing control data which can be transmitted via the supply line (102), **characterized in that** the module (112, 113.1, 113.2, 113.3, 113.n) is arranged on the slide.

2. Device according to Claim 1, **characterized in that** the data can be transmitted in the form of an electrical signal via the supply line (102) at a frequency of greater than 100 Hz, the frequency being, in particular, higher than 1 kHz, preferably in the range of 50 kHz to 2 MHz.

3. Device according to either of Claims 1 and 2, **characterized in that** the communication device comprises a coupler which can be used to couple data into the supply line (102) and/or out of the supply line (102).

4. Device according to one of Claims 1 to 3, **characterized in that** the data can be transmitted via the supply line (102) by means of frequency shift keying.

5. Device according to one of Claims 1 to 4, **characterized in that** the data comprise control data for controlling the module (112, 113.1, 113.2, 113.3, 113.n) and/or measurement data provided by the module (112, 113.1, 113.2, 113.3, 113.n).

6. Device according to one of Claims 1 to 5, **characterized in that**, in order to be connected to an external energy source, it comprises a power connection, to which the supply line (102) is connected, and the device preferably comprises a low-pass filter (105) which can be either inserted into the supply line (102) or removed from the latter using a switching device (106).

7. Device according to Claim 6, **characterized in that** it comprises a transformer (104) which is connected into the supply line (102) between the power connection and the communication device (110).

8. Device according to Claim 7, **characterized in that** the module (112, 113.1, 113.2, 113.3, 113.n) comprises the transformer (104).

9. Device according to one of Claims 1-8, **characterized in that** the module (112, 113.1, 113.2, 113.3, 113.n) comprises a lighting device (111.1-111.4).

10. Device according to Claim 9, **characterized in that** the lighting device (111.1-111.4) comprises at least one LED (111.4) and is designed, in particular, to produce slit lighting (111.1).

11. Device according to either of Claims 9 and 10, **characterized in that** it comprises a light guide (234) for ambient lighting which branches off from the lighting device (111.1-111.4).

12. Device according to one of Claims 1 to 11, **characterized in that** the module (112, 113.1, 113.2, 113.3, 113.n) comprises a display device.

13. Device according to one of Claims 1 to 12, **characterized in that** the module (112, 113.1, 113.2, 113.3, 113.n) comprises a memory unit and a computing unit (111), the memory unit being able to store a program and the program being able to be executed by the computing unit and being able to be controlled using data received via the supply line (102).

## Revendications

1. Dispositif pour examiner et/ou traiter un oeil (100), comprenant un chariot pouvant se déplacer dans plusieurs directions dans l'espace, un microscope disposé sur le chariot et une ligne d'alimentation (102) ainsi qu'un module (112, 113.1, 113.2, 113.3, 113.n), lequel est relié à la ligne d'alimentation (102) pour être alimenté en énergie électrique, le module (112, 113.1, 113.2, 113.3, 113.n) étant relié à un dispositif de commutation (110) raccordé à la ligne d'alimentation (102) pour envoyer et/ou recevoir des données par le biais de la ligne d'alimentation (102), un élément électrique, électronique et/ou mécanique commandable et une commande munie d'éléments de commande (116 ; 216) servant à fournir des données de commande pouvant être transmises par le biais de la ligne d'alimentation (102), **caractérisé en ce que** le module (112, 113.1, 113.2, 113.3, 113.n) est monté sur le chariot.

2. Dispositif selon la revendication 1, **caractérisé en ce que** les données peuvent être transmises par le biais de la ligne d'alimentation (102) sous la forme d'un signal électrique ayant une fréquence supérieure à 100 Hz, la fréquence étant notamment supérieure à 1 kHz, de préférence comprise dans la plage de 50 kHz à 2 MHz.

3. Dispositif selon l'une des revendications 1 à 2, **caractérisé en ce que** le dispositif de communication comprend un coupleur avec lequel les données dans la ligne d'alimentation (102) peuvent être injectées dans et/ou extraites hors de la ligne d'alimentation (102).

4. Dispositif selon l'une des revendications 1 à 3, **caractérisé en ce que** les données peuvent être transmises par modulation par déplacement de fréquence sur la ligne d'alimentation (102).

5. Dispositif selon l'une des revendications 1 à 4, **caractérisé en ce que** les données comprennent des données de commande destinées à commander le module (112, 113.1, 113.2, 113.3, 113.n) et/ou des données de mesure délivrées par le module (112, 113.1, 113.2, 113.3, 113.n).

6. Dispositif selon l'une des revendications 1 à 5, **caractérisé en ce qu'**il comprend une borne électrique destinée au raccordement à une source d'énergie externe, avec laquelle est reliée la ligne d'alimentation (102), et le dispositif comprend de préférence un filtre passe-bas (105) qui peut être inséré dans la ligne d'alimentation (102) ou, au choix, en être déconnecté par un dispositif de commutation (106).

7. Dispositif selon la revendication 6, **caractérisé en ce qu'**il comprend un transformateur (104) qui est branché dans la ligne d'alimentation (102) entre la borne électrique et le dispositif de communication (110).

8. Dispositif selon la revendication 7, **caractérisé en ce que** le module (112, 113.1, 113.2, 113.3, 113.n) comprend le transformateur (104).

9. Dispositif selon l'une des revendications 1 à 8, **caractérisé en ce que** le module (112, 113.1, 113.2, 113.3, 113.n) comprend un dispositif d'éclairage (111.1 - 111.4).

10. Dispositif selon la revendication 9, **caractérisé en ce que** le dispositif d'éclairage (111.1 - 111.4) comprend au moins une LED (111.4) et est notamment configuré pour générer un éclairage de fente (111.1).

11. Dispositif selon l'une des revendications 9 à 10, **caractérisé en ce qu'**il comprend une fibre optique (234) se détachant du dispositif d'éclairage (111.1 - 111.4) pour un éclairage périphérique.

12. Dispositif selon l'une des revendications 1 à 11, **caractérisé en ce que** le module (112, 113.1, 113.2, 113.3, 113.n) comprend un dispositif d'affichage.

13. Dispositif selon l'une des revendications 1 à 12, **caractérisé en ce que** le module (112, 113.1, 113.2, 113.3, 113.n) comprend une unité de mémoire et une unité de calcul (111), un programme pouvant être enregistré dans l'unité de mémoire et le programme pouvant être exécuté par l'unité de calcul et pouvant être commandé au moyen de données reçues par le biais de la ligne d'alimentation (102).
